# EUROPEAN PATENT APPLICATION

(11) **EP 4 559 454 A1**
(43) Date of publication of application: **28.05.2025**
(21) Application number: 24214097.8
(22) Date of filing: 20.11.2024
(51) Int. Cl.: A61K 9/00, A61K 9/20

(54) **ORALLY DISINTEGRATING TABLET FORMULATIONS OF BRIVARACETAM**

(30) Priority: 22.11.2023 TR 202315499
(71) Applicant: Sanovel Ilac Sanayi ve Ticaret A.S., Sisli - Istanbul (TR)
(72) Inventor: SUNEL, Fatih, Istanbul (TR); BILGEHAN ATAK, Fadime, Istanbul (TR); ATAMAN, Seval, Istanbul (TR); DERELI, Selin, Istanbul (TR)
(74) Representative: Genç Ilhan, Oznur

(57) **Abstract**

The present invention relates to an orally disintegrating tablet formulation comprising brivaracetam or a pharmaceutically acceptable salt thereof and at least one pharmaceutically acceptable excipient wherein at least one sugar derivative as diluent. The present invention also relates to a simple, rapid, cost effective, time-saving and industrially convenient process of preparing the composition.

## Description

### Field of the Invention

The present invention relates to an orally disintegrating tablet formulation comprising brivaracetam or a pharmaceutically acceptable salt thereof and at least one pharmaceutically acceptable excipient wherein at least one sugar derivative as diluent. The present invention also relates to a simple, rapid, cost effective, time-saving and industrially convenient process of preparing the composition.

### Background of the Invention

The chemical name of brivaracetam is (2S)-2-[(4R)-2-oxo-4-propyl-pyrrolidin-1-yl]butanamide and its chemical structure is shown in the Formula I.

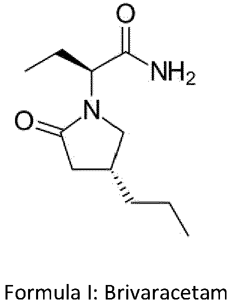

Brivaracetam is a white to off-white crystalline powder. It is very soluble in water, buffer (pH 1.2, 4.5 and 7.4), ethanol, methanol, and glacial acetic acid. It is freely soluble in acetonitrile and acetone and soluble in toluene. It is very slightly soluble in n-hexane.

Brivaracetam is commercially approved in the form of tablets, oral solution and injection, in the USA under the brand name Briviact and are marketed by UCB Pharma.

Over the past decades, orally disintegrating tablets (ODTs) have gained considerable attention as a preferred alternative to conventional tablets and capsules due to better patient compliance. ODTs are solid dosage forms containing active ingredients which disintegrate rapidly through buccal mucosa. It is desirable in the treatment of a number of diseases. Particularly, in epilepsy treatment in which it is very crucial to use drug on time and properly as described by the doctor. They are also advantageous for administrations of medicaments to patients who are traveling or have little access to water are similarly affected or patients who are mentally retarded, uncooperative, nauseated, or patients who have difficulties swallowing other dosage forms.

It is difficult to develop orally disintegrating tablet because of several different reasons and requirements such as disintegration, stability and taste masking properties. Dosage form must disintegrate in the oral cavity with the existence of saliva in a short period of time. So, those compositions should have a porous structure. However, these porous characteristics tend to be very sensitive to humidity and may be leads to stability problems. Moreover, effective taste masking technologies should also be adopted for bitter taste of active ingredient.

To fulfill all these requirements, the oral disintegrating tablet formulation for brivaracetam needs to be adapted in particular by a careful selection of the excipients which give a high porous structure with high stability and suitable disintegration time. Additionally, to overcome bitter taste problem, taste masking has to be taken into consideration during the process.

### Detailed Description of the Invention

The main object of the present invention is to provide an oral disintegrating tablet (ODT) is safe to take and good disintegrating property and good mouth feel.

Another object of the present invention is to provide a ODT comprising brivaracetam has the desired stability.

Another object of the present invention is to provide a preparation process of the oral disintegrating tablet comprising brivaracetam which is simple and cost-effective process.

As orally disintegrating tablet is designed to rapidly disintegrate as the drug comes in direct contact with the tongue, it remains a challenge to the formulators to effectively mask the taste of bitter drugs such as brivaracetam inhibitors to increase the acceptability for these compositions.

The present invention provides an orally disintegrating tablet formulation comprising brivaracetam or a pharmaceutically acceptable salt thereof and at least one pharmaceutically acceptable excipient wherein at least one sugar derivative as diluent.

Taste is one of the most important parameters governing patient compliance for ODT formulations. With an excipient that is a sugar derivative, we have achieved a formulation that provides the desired dissolution profile and gives the tablet a good taste. Thus, by using these excipients that have both a sweetening effect and diluent properties, we have obtained tablets with the desired dissolution profile and the desired porous structure and also high patient compliance, in an easy and convenient way. Surprisingly, furthermore it was found according to the invention that the stability of the ODT, as defined above, can be achieved with substances which have a basic structure based on sugar derivatives.

According to this embodiment of the present invention, sugar derivatives is selected from the group comprising fructose, mannitol, lactose, sorbitol, xylitol, maltodextrin, dextrates, dextrins, lactitol and mixtures thereof.

According to this embodiment of the present invention, fructose is used as a diluent in the ODT formulation.

According to this embodiment of the present invention, the amount of fructose is between 1.0% and 30.0% by weight in the total formulation. Preferably, the amount of fructose is between 3.0% and 20.0% by weight in the total formulation.

According to this embodiment of the present invention, mannitol is used as a diluent in the ODT formulation.

According to this embodiment of the present invention, the amount of mannitol is between 25.0% and 75.0% by weight in the total formulation. Preferably, the amount of mannitol is between 35.0% and 70.0% by weight in the total formulation.

According to this embodiment of the present invention, the amount of brivaracetam is between 5.0% and 35.0% by weight in the total formulation. Preferably, the amount of brivaracetam is between 7.0% and 30.0% by weight in the total formulation.

According to this embodiment of the present invention, the orally disintegrating tablet formulation further comprises at least one pharmaceutically acceptable excipient is selected from the group comprising disintegrants, diluents, lubricants, glidant, sweetener, flavoring agent or mixtures thereof.

Suitable disintegrants are selected from the group comprising crospovidone, starch, croscarmellose sodium, low-substituted hydroxypropyl cellulose, sodium starch glycolate, carboxymethyl cellulose, sodium carboxymethyl cellulose, calcium carboxymethyl cellulose, sodium carboxymethyl starch, hydroxymethyl starch or mixtures thereof.

According to an embodiment of the present invention, the disintegrant is crospovidone or starch or mixtures thereof.

According to this embodiment of the present invention, the amount of disintegrant is between 5.0% and 25.0% by weight in the total formulation. Preferably, the amount of disintegrant is between 7.0% and 20.0% by weight in the total formulation. Its use in this range provided the desired dissolution profile. We also found that this range ensures content uniformity of the active ingredient.

Suitable diluents are selected from the group comprising microcrystalline cellulose, talc, calcium hydrogen phosphate dihydrate, dicalcium hydrogen phosphate anhydrate, calcium phosphate trihydrate, neutral pellets, magnesium carbonate, magnesium oxide, maltose, medium chain triglycerides or mixtures thereof.

According to an embodiment of the present invention, the diluent is microcrystalline cellulose.

According to this embodiment of the present invention, the amount of microcrystalline cellulose is between 5.0% and 30.0% by weight in the total formulation. Preferably, the amount of microcrystalline cellulose is between 7.0% and 25.0% by weight in the total formulation.

Suitable glidants are selected from the group comprising colloidal silicon dioxide (Aerosil), colloidal anhydrose silica, aluminum silicate, powdered cellulose, calcium phosphate tribasic, hydrophobic colloidal silica, magnesium trisilicate, magnesium silicate or mixtures thereof.

In one embodiment, the glidant is colloidal silicon dioxide.

According to this embodiment of the present invention, the amount of colloidal silicon dioxide is between 0,1% and 5.0% by weight in the total formulation.

Suitable lubricants are selected from the group comprising sodium stearyl fumarate, magnesium stearate, sodium lauryl sulphate, zinc stearate, calcium stearate, mineral oil, polyethylene glycol, glyceryl monostearate, glyceryl palmitostearate, magnesium lauryl sulphate, stearic acid, hydrogenated natural oils, silica, paraffin or mixtures thereof.

According to an embodiment of the present invention, the lubricant is sodium stearyl fumarate or magnesium stearate or mixtures thereof.

According to this embodiment of the present invention, the amount of lubricant is between 0.1% and 5.0% by weight in the total formulation.

According to an embodiment of the present invention, flavoring agent can use in the ODT formulation. Suitable flavoring agents are selected from the group comprising Lemon flavor, menthol, peppermint, cinnamon, chocolate, vanillin, or fruit essences such as cherry, orange, strawberry, grape, black currant, raspberry, banana, red fruits, wild berries or mixtures thereof.

In one embodiment, the flavoring agent is lemon flavor.

According to this embodiment of the present invention, the amount of lemon flavor is between 0,5% and 10.0% by weight in the total formulation.

Suitable sweeteners may include but not limited to sucralose, aspartame, erythritol, thaumatin, mogroside, inulin, acesulfame-K, saccharin or its sodium and calcium salts, sodium cyclamate, sucrose, glucose, menthol, peppermint, cinnamon, chocolate, vanillin, and fruit essences such as cherry, orange, strawberry, grape or mixtures thereof.

According to an embodiment of the present invention, the sweeteners is sucralose or aspartam or mixtures thereof.

According to this embodiment of the present invention, the amount of sweetener is between 0,1% and 5.0% by weight in the total formulation.

As used here in, 'particle size' means the cumulative volume size distribution as tested by any conventionally accepted method such as the laser diffraction method (i.e. Malvern analysis). The term d (0.1) means, the size at which 10% by volume of the particles are finer and d (0.5) means the size at which 50% by volume of the particles are finer and d (0.9) means the size at which %90 by volume of the particles is finer.

According to one embodiment of this invention, brivaracetam has a d (0.9) particle size between 30 µm to 450 µm. These particle ranges helped provide the desired dissolution profile.

According to one embodiment of this invention, brivaracetam has a d (0.9) particle size between 50 µm to 90 µm or between 95 µm to 125 µm or between 126 µm to 138 µm or between 139 µm to 152 µm or between 153 µm to 167 µm or between 168 µm to 183 µm or between 184 µm to 198 µm or between 203 µm to 218 µm or between 219 µm to 234 µm or between 237 µm to 253 µm or between 257 µm to 268 µm or between 269 µm to 278 µm or between 283 µm to 298 µm or between 304 µm to 345 µm or between 348 µm to 390 µm or between 395 µm to 445 µm.

According to one embodiment of this invention, brivaracetam has a d (0.1) particle size between 1 µm to 40 µm or between 4 µm to 34 µm or between 6 µm to 25 µm.

According to one embodiment of this invention, brivaracetam has a d (0.5) particle size between 5 µm to 85 µm or between 13 µm to 76 µm or between 22 µm to 65 µm or between 27 µm to 58 µm.

In direct compression, the solid pharmaceutical compositions ingredients are not exposed to moisture, solvents and heat. Thus, in this invention, direct compression is used to process moisture, solvent and/or heat sensitive brivaracetam. In addition, this process provides the desired compressibility and flowability without loss of active substance. We made this application using the direct compression process. Due to the sensitivity of the active ingredient to moisture, we made it with the simplest process without using solvents. The production process has been successfully completed in a way that does not shatter with easy physical impacts but can easily disintegrate in the mouth.

According to one embodiment of this invention, the orally disintegrating tablet is obtained by direct compression.

### Example 1: Direct compression

| **Ingredients** | **% by weight of the total tablet** | **% by weight of the total core tablet** | **% by weight of the total core tablet** |
|---|---|---|---|
| Brivaracetam | 5.0 - 35.0 | 10 | 25 |
| Mannitol | 25-50 | 43,5 | 38,5 |
| Microcrystalline cellulose | 5-25 | 20 | 10 |
| Fructose | 5-20 | 13 | 13 |
| Sucralose | 0,1-5 | 1 | 1 |
| Crospovidone | 5-20 | 10 | 10 |
| Colloidal silicon dioxide | 0,1-5 | 1,5 | 1,5 |
| Sodium stearyl Fumarate | 0,1-5 | 1 | 1 |
| **TOTAL TABLET** | **100** | **100** | **100** |

### Example 1;

The pharmaceutical compositions mentioned above are prepared by following these steps:
1) Weigh and sieve Brivaracetam, Mannitol, Microcrystalline cellulose, Fructose, Sucrolose, Crospovidone, Colloidal silicon dioxide and mix for 15 minutes.
2) Add weighed and 0.6 mm sieved sodium stearyl fumarate to dry mixture and mix for 3 minutes.
3) Tablet the homogenous mixture following appropriate specifications gain.

### Example 2: Direct compression

| **Ingredients** | **% by weight of the total core tablet** | **% by weight of the total core tablet** | **% by weight of the total core tablet** |
|---|---|---|---|
| Brivaracetam | 5.0 - 35.0 | 10 | 25 |
| Mannitol | 45-75 | 67 | 55 |
| Starch | 5-25 | 16.75 | 13.75 |
| Colloidal Silicon dioxide | 0,1-5 | 1.25 | 1.25 |
| Aspartam | 0,5-5 | 1.25 | 1.25 |
| Lemon flavor | 0,1-5 | 2.5 | 2.5 |
| Magnesium stearate | 0,1-5 | 1.25 | 1.25 |
| **TOTAL TABLET** | **100** | **100** | **100** |

### Example 2;

The pharmaceutical compositions mentioned above are prepared by following these steps:
1) Weigh and sieve Brivaracetam, Mannitol, Starch, Aspartam, lemon flavor, colloidal silicon dioxide and mix for 15 minutes.
2) Add weighed and 0.6 mm sieved Magnesium stearate to dry mixture and mix for 3 minutes.
3) Tablet the homogenous mixture following appropriate specifications gain.

## Claims

1. An orally disintegrating tablet formulation comprising brivaracetam or a pharmaceutically acceptable salt thereof and at least one pharmaceutically acceptable excipient wherein at least one sugar derivative as diluent.

2. The orally disintegrating tablet formulation according to claim 1, wherein sugar derivatives are selected from the group comprising fructose, mannitol, lactose, sorbitol, xylitol, maltodextrin, dextrates, dextrins, lactitol and mixtures thereof.

3. The orally disintegrating tablet formulation according to claim 1 or 2, wherein diluent is fructose.

4. The orally disintegrating tablet formulation according to claim 1or 2, wherein diluent is mannitol.

5. The orally disintegrating tablet formulation according to claim 1, wherein further comprises at least one pharmaceutically acceptable excipient is selected from the group comprising disintegrants, diluents, lubricants, glidant, sweetener, flavoring agent or mixtures thereof.

6. The orally disintegrating tablet formulation according to claim 5, wherein disintegrants are selected from the group comprising crospovidone, starch, croscarmellose sodium, low-substituted hydroxypropyl cellulose, sodium starch glycolate, carboxymethyl cellulose, sodium carboxymethyl cellulose, calcium carboxymethyl cellulose, sodium carboxymethyl starch, hydroxymethyl starch or mixtures thereof.

7. The orally disintegrating tablet formulation according to claim 6, wherein disintegrant is crospovidone or starch or mixtures thereof.

8. The orally disintegrating tablet formulation according to claim 5, wherein glidants are selected from the group comprising colloidal silicon dioxide (Aerosil), colloidal anhydrose silica, aluminum silicate, powdered cellulose, calcium phosphate tribasic, hydrophobic colloidal silica, magnesium trisilicate, magnesium silicate or mixtures thereof.

9. The orally disintegrating tablet formulation according to claim 8, wherein glidant is colloidal silicon dioxide.

10. The orally disintegrating tablet formulation according to claim 5, wherein lubricants are selected from the group comprising sodium stearyl fumarate, magnesium stearate, sodium lauryl sulphate, zinc stearate, calcium stearate, mineral oil, polyethylene glycol, glyceryl monostearate, glyceryl palmitostearate, magnesium lauryl sulphate, fumaric acid, stearic acid, hydrogenated natural oils, silica, paraffin or mixtures thereof.

11. The orally disintegrating tablet formulation according to claim 10, wherein lubricant is sodium stearyl fumarate or magnesium stearate or mixtures thereof.

12. The orally disintegrating tablet formulation according to claim 5, wherein flavoring agents are selected from the group comprising Lemon flavor, menthol, peppermint, cinnamon, chocolate, vanillin, or fruit essences such as cherry, orange, strawberry, grape, black currant, raspberry, banana, red fruits, wild berries or mixtures thereof.

13. The orally disintegrating tablet formulation according to claim 12, wherein flavoring agent is lemon flavor.

14. The orally disintegrating tablet formulation according to claim 5, wherein sweeteners may include but not limited to sucralose, aspartame, erythritol, thaumatin, mogroside, inulin, acesulfame-K, saccharin or its sodium and calcium salts, sodium cyclamate, sucrose, glucose, menthol, peppermint, cinnamon, chocolate, vanillin, and fruit essences such as cherry, orange, strawberry, grape or mixtures thereof.

15. The orally disintegrating tablet formulation according to claim 14, wherein sweetener is sucralose or aspartam or mixtures thereof.
